# EUROPEAN PATENT SPECIFICATION

(11) **EP 1 804 807 B1**
(45) Date of publication and mention of the grant of the patent: **03.12.2008**
(21) Application number: 05851251.8
(22) Date of filing: 24.10.2005
(51) Int. Cl.: A61K 31/65, A61K 31/585, A61P 9/04

(54) **COADMINISTRATION OF TIGECYCLINE AND DIGOXIN**
GEMEINSAME GABE VON TIGECYCLIN UND DIGOXIN
CO-ADMINISTRATION DE TIGECYCLINE ET DE DIGOXINE

(30) Priority: 28.10.2004 US 622859 P
(43) Date of publication of application: 11.07.2007
(73) Proprietor: Wyeth, Madison, NJ 07940 (US)
(72) Inventor: RAIBLE, Donald, G., Devon, Pennsylvania 19333 (US); MURALIDHARAN, Gopal, Bangalore 560 070 (IN)
(74) Representative: Talbott, Dawn Jacqueline
(86) International application number: PCT/US2005/038410
(87) International publication number: WO 2006/060084

(56) References cited:
- ZIMMERMAN J J ET AL: "Tigecycline (Tgc) and digoxin (Dig) co-administered to healthy men" ABSTRACTS OF THE INTERSCIENCE CONFERENCE OF ANTIMICROBIAL AGENTS AND CHEMOTHERAPY, vol. 44, October 2004 (2004-10), page 18, XP001246594
- MEAGHER ET AL: "Pharmacokinetic/pharmacodynamic profile for tigecycline-a new glycylcycline antimicrobial agent" DIAGNOSTIC MICROBIOLOGY AND INFECTIOUS DISEASES, ELSEVIER SCIENCE PUBLISHING CO., AMSTERDAM, NL, vol. 52, no. 3, July 2005 (2005-07), pages 165-171, XP005026753 ISSN: 0732-8893
- STAHL J-P: "Tigecycline: a new antibiotic in ongoing clinical development" MEDECINE ET MALADIES INFECTIEUSES, SOCIETE FRANCAISE D'EDITIONS MEDICALES, PARIS, FR, vol. 35, no. 2, February 2005 (2005-02), pages 62-67, XP004849240 ISSN: 0399-077X
- SMITH K L ET AL: "Tigecycline: A novel antibiotic"[Online] 1 August 2005 (2005-08-01), XP002377021 Retrieved from the Internet: URL:http://mediwire.skyscape.com/main/Defa ult.aspx?P=Content&ArticleID=177008> [retrieved on 2006-04-12]
- ANONYMOUS: "PDR Drug information for TIGECYCLINE" DRUGS.COM - DRUG INFORMATION ONLINE, [Online] June 2005 (2005-06), XP002377022 Retrieved from the Internet: URL:http://www.drugs.com/pdr/TIGECYCLINE.h tml> [retrieved on 2006-04-12]
- DERUITER J ET AL: "New Anti-Infective Drugs of 2005" US PHARMACIST, [Online] 1 October 2005 (2005-10-01), XP002377023 Retrieved from the Internet: URL:http://www.uspharmacist.com/index.asp? page=ce/105130/default.htm> [retrieved on 2006-04-12]

## Description

### FIELD OF THE INVENTION

The invention relates to treatment of bacterial infections with tigecycline and cardiac insufficiency with digoxin by coadministration to a human in need thereof.

### BACKGROUND OF THE INVENTION

Tigecycline (GAR-936) is a glycylcycline antibiotic and an analog of the semisynthetic tetracycline, minocycline. Tigecycline has broad-spectrum antibacterial activity both in vitro and in vivo. Further, tigecycline was developed in response to the worldwide threat of emerging resistance to antibiotics. Glycylcycline antibiotics, like tetracycline antibiotics, act by inhibiting protein translation in bacteria.

Glycylcyclines, including tigecycline, are active against many antibiotic-resistant gram-positive pathogenic bacteria, such as methicillin-resistant *Staphylococcus aureus*, penicillin-resistant *Streptococcus pneumoniae*, and vancomycin-resistant enterococci (Weiss et al., 1995; Fraise et al., 1995). Of great significance is the activity of tigecycline against bacterial strains carrying the two major forms of tetracycline resistance, efflux and ribosomal protection (Schnappinger and Hillen, 1995).

Digoxin is a digitalis glycoside inotropic drug used extensively to treat cardiac insufficiency. However, it is likely that individuals in the critical care setting who require intravenous antiinfective therapy could also already be receiving or begin receiving digoxin for a coexisting cardiac condition. A major clinical concern surrounding coadministration of other drugs, in particular antiinfectives with digoxin is one of cardiac toxicity resulting from increased plasma levels of digoxin in a patient with preexisting cardiac insufficiency. This is of grave concern since digoxin has a very narrow therapeutic index. For example, Clarithromycin in particular has been shown to increase plasma levels of digoxin, sometimes to toxic levels, (Xu H, Rashkow A. Clarithromycin-induced digoxin toxicity: a case report and a review of the literature. Connecticut Medicine 2001;65:527-9; and Gooderham MJ, Bolli P, Fernandez PG. Concomitant digoxin toxicity and warfarin interaction in a patient receiving clarithromycin. Annals of Pharmacotherapy 1999;33:796-9); this interaction has been linked to reduced renal clearance of digoxin (Rengelshausen J, Goggelmann C, Burhenne J, Riedel KD, Ludwig J, Weiss J, et al. Contribution of increased oral bioavailability and reduced nonglomerular renal clearance of digoxin to the digoxin-clarithromycin interaction. British Journal of Clinical Pharmacology 2003;56:32; Baron JM, Goh LB, Yao D, Wolf CR, Friedberg T. Modulation of P450 CYP3A4-dependent metabolism by P-glycoprotein: implications for P450 phenotyping. Journal of Pharmacology & Experimental Therapeutics 2001;296:351-8; Nordt SP, Williams SR, Manoguerra AS, Clark RF, Clarithromycin induced digoxin toxicity, Journal of Accident & Emergency Medicine 1998; 15:194-5; Tanaka H, Matsumoto K, Ueno K, Kodama M, Yoneda K, Katayama Y, et al. Effect of clarithromycin on steady-state digoxin concentrations. Annals of Pharmacotherapy 2003;37:178-81; and Wakasugi H, Yano I, Ito T, Hashida T, Futami T, Nohara R, et al. Effect of clarithromycin on renal excretion of digoxin: interaction with P-glycoprotein.Clinical Pharmacology & Therapeutics 1998;64:123-8, which, in tum, may be linked to P-gp transport (Rengelshausen J, Goggelmann C, Burhenne J, Riedel KD, Ludwig J, Weiss J, et al. Contribution of increased oral bioavailability and reduced nonglomerular renal clearance of digoxin to the digoxin-clarithromycin interaction. British Journal of Clinical Pharmacology 2003;56:32-8).

Tetracycline and minocycline interaction with digoxin has been described in the reference Roos T C and Merk H F, Drugs, (2000) 59/2 (181-192). Interactions of tetracyclines are also described by Gregg C R, Am.J.Med. (106, No. 2, 227-37, 1999). The interaction of digoxin with quinidine, verapamil, & of p.o. digoxin with broad-spectrum antibiotics such as erythromycin or tetracycline HCl is discussed in the reference Roffman D S, Postgraduate Medicine, (1997).

There is therefore a need for a combination of an antibiotic and digoxin that addresses the problems noted above especially increased plasma digoxin levels and the toxicity as a result of the same since digoxin is a drug with a very narrow therapeutic index

### BRIEF DESCRIPTION OF THE DRAWINGS

FIG. 1 Mean (SE) Plasma Digoxin Concentrations, hours 0 through 24. Period 2 (Digoxin alone, 0.25 mg/day + tigecycline 50 mg/12h) Versus Period 3 (tigecycline 50 mg/12h + digoxin 0.25 mg/day)
FIG. 2 Mean (SE) Serum Tigecycline Concentrations: Hours 0 through 12 Period 1 (Tigecycline alone, 100 mg Single Dose) Versus Period 3 and 3 (Tigecycline 50 mg/12h + Digoxin 0.25 mg/day)
FIG. 3 Mean (SE) Serum Tigecycline Concentrations, Hours 0 through 96, Period 1 (Tigecycline Alone, 100 mg Single Dose) Versus Period 3 (Tigecycline 50/12h + Digoxin 0.25 mg/day)
FIG. 4 Distribution of ECG changes for predose values in QT interval in healthy subjects, Tigecycline alone, digoxin alone, digoxin + Tigecycline concomitantly

### BRIEF SUMMARY OF THE INVENTION

The invention relates to the coadministration of tigecycline and digoxin to a human patient without the condition of cardiac compromise resulting from increased plasma levels of digoxin and potential toxicity in said patient with preexisting cardiac insufficiency.

The invention further relates to the preparation of a medicament for use in a method of treating, controlling or reducing the risk of a bacterial infection and a cardiac insufficiency condition in a human which comprises administering to said human in need thereof an effective amount of tigecycline and an effective amount of digoxin.

The invention further relates to the preparation of a medicament for use in a method for improving steady state digoxin plasma levels in a human experiencing cardiac insufficiency condition and a bacterial infection, the method comprising administering to said human in need thereof an effective amount of digoxin and tigecycline.

The invention relates to the preparation of a medicament for use in a method of treating, controlling or reducing the risk of a bacterial infection and a cardiac insufficiency condition in a human which comprises administering to said human in need thereof an effective amount of tigecycline and an effective amount of digoxin.

The invention relates to the preparation of a medicament for use in a method of treating, controlling or reducing the risk of a bacterial infection with tigecycline in a patient having preexisting cardiac insufficiency and being treated with digoxin said method having the advantage of controlling and stabilizing from increasing plasma digoxin levels in said patient.

The invention relates to the preparation of a medicament for use in a method of treating, controlling or reducing the risk of a cardiac insufficiency condition and a bacterial infection in a human which comprises administering to said human in need thereof an effective amount of digoxin and an effective amount of tigecycline.

Following intravenous administration of tigecycline and oral administration of digoxin to healthy, male volunteers, an analysis was performed to determine by pharmacokinetic (PK) and pharmacodynamic (PD) assessments the absence of any clinically significant interaction.

It was determined that by treating humans by intravenous (IV) infusion of tigecycline in 0.09% sterile normal saline over 30 minutes and administering digoxin orally with 240 ml of room-temperature water that digoxin and tigecycline may be coadministered.

The overriding clinical concern surrounding coadministration of tigecycline and digoxin is one of cardiac compromise resulting from increased plasma levels of digoxin in a patient with preexisting cardiac insufficiency. From pharmacokinetic and bioequivalence viewpoints, the following described clinical results suggest that the coadministration of tigecycline would not effect such a compromise. Specifically, tigecycline did not affect the steady-state plasma digoxin AUC0-24h, CL/F, or digoxin concentrations during the 12- to 24-hour period after dose administration (therapeutic drug monitoring times), although the 90% Cls for Cmax and tmax fell outside of the equivalence window. Tigecycline also did not affect the steady-state digoxin urinary PK as shown by measurement of digoxin Ae,% and digoxin CLr. Another concern with coadministering these 2 drugs is a potential compromise in therapeutic serum tigecycline concentrations in a patient being treated for a complicated infection in the critical care setting. Although digoxin increased both tigecycline t1/2 and Vss, these increases did not affect tigecycline AUC or CL; hence, tigecycline exposure during the concomitant administration of digoxin would probably be unchanged, necessitating no tigecycline dosage adjustment in a patient receiving a therapeutic dosage of digoxin.

The present invention provides to the art a new method useful for the treatment or control of bacterial infections by parenteral administration, and oral coadministered with digoxin which avoids adverse interactions.

Other advantages and aspects of the present invention will become apparent upon reading the following detailed description of the invention.

### DESCRIPTION OF PREFERRED EMBODIMENTS

The following definitions are used throughout the application.

"Cardiac insufficiency condition" or "cardiac insufficiency" means slow failure of the heart and occurs when the heart loses its ability to pump enough blood through the body. Further is also any condition which calls for the use of digoxin which includes preexisting cardiac insufficiency.

"Treating" refers to reversing, alleviation of symptoms or inhibiting the progress of a bacterial infection. Further, treating also means reducing and alleviation of symptoms and conditions associated with cardiac insufficiency with digoxin.

"Administering" means a treatment process wherein an effective amount of tigecycline is delivered to a human patient. Further, administering means a treatment process wherein an effective amount of digoxin is delivered to a human patient.

"Bacterial infection" is the proliferation of a bacteria pathogen caused by Gram-positive and Gram-negative bacteria.

"Effective amount" is an amount of tigecycline, where upon administration, is capable of reducing or preventing the proliferation of bacteria or reducing the symptoms of the bacterial infection. Further, effective amount means an amount of digoxin capable of reducing or preventing cardiac insufficiency condition. Additionally, the effective amount means an amount of tigecycline which will not increase the Cmax of digoxin.

"Coadministration" is simultaneous or sequential coadministration of tigecycline and, digoxin. When administration is sequential, either the tigecycline or the digoxin may be administered first.

### EXPERIMENTAL METHODS

### MATERIALS AND METHODS

### Study subjects

Healthy men aged 27 to 45 years who were in good health on the basis of medical history, physical examination, electrocardiograms (ECGs), and laboratory evaluations, and had a body mass index in the range of 18 to 30 kg/m2 and body weight ≥ 50 kg, were enrolled. Subjects were nonsmokers or smoker of fewer than 10 cigarettes (half a pack) per day as determined by history and able to abstain from smoking during the inpatient stay.

Tobacco use or the consumption of any caffeine-containing products (eg, coffee, tea, chocolate, or cola), grapefruit, grapefruit-containing products, or alcoholic beverages was prohibited from at least 48 hours before study day 1 until the end of the inpatient confinement period.

Subjects were excluded if they had a history or presence of any significant cardiovascular (including Wolf-Parkinson-White syndrome), hepatic, renal, respiratory, gastrointestinal, endocrine, immunologic, dermatologic, hematologic, neurologic, or neuropsychiatric disease, surgical or other medical condition that may have interfered with the absorption, distribution, metabolism, or excretion of either study drug, acute disease state (eg, nausea, vomiting, fever, diarrhea) within 7 days of study day 1, admitted alcohol abuse or consumption of more than 2 standard units per day, any clinically important deviation from normal limits in physical examination, vital signs, or clinical laboratory test results, positive serologic findings for HIV antibodies, hepatitis B or C surface antigen and/or antibodies, positive drug screen (eg, amphetamines, barbiturates, benzodiazepines, cannabinoids, cocaine, opiates), or had a PR interval ≥200 msec; resting heart rate ≥50 bpm at screening or on day-1.

The study was conducted at the Wyeth Clinical Pharmacology Unit, Philadelphia, PA, USA, and was approved by the Institutional Review Board of The Methodist Hospital in Philadelphia, PA, USA, and was conducted according to the Declaration of Helsinki and its amendments. All subjects gave written informed consent before enrollment.

### Study medications

Tigecycline (Wyeth Pharmaceuticals, Collegeville, PA, USA) was supplied as lyophilized powder in 5-mL, flint-glass vials, each containing lyophilized free base equivalent to 50 mg of tigecycline without additives or preservatives. This powder was reconstituted with sterile normal saline (0.9% NaCl for Injection, USP) to the correct volume before administration. Digoxin was supplied as Lanoxin^{®} (Glaxo SmithKline, Collegeville, PA, USA) 0.25 mg tablets for oral administration.

### Study design and treatment

The purpose of this open-label, single-sequence, 3-period, multiple-dose crossover drug interaction study was to determine the effects of steady-state tigecycline concentrations on steady-state levels of digoxin. The coadministration of multiple doses of digoxin and tigecycline maximized the potential to detect an interaction. Because this was a single-sequence crossover study, multiple washout periods were not necessary; this was an important consideration because both digoxin and tigecycline have long half-lives (t_{1/2}).

A 20% or greater difference in the area under the plasma concentration-time curve during a dose interval (AUC_{0-T}) of digoxin could be considered a clinically significant interaction. With a sample size of 16, the statistical power for detecting a 20% difference in AUC_{0-T} at a 0.05 level of significance was expected to exceed 80%.

On each day before the start of study periods 1 and 2 (day -1 and day 6), all subjects underwent physical examinations, laboratory tests, vital sign assessments, and a standard 12-lead electrocardiogram (ECG), which included measurements of rhythm, heart rate, PR, QRS, QT, and QTc intervals. Adverse event monitoring was continuous, and blood samples for PK analysis was completed at the designated times throughout all study periods. Before dose administration on days 1, 7 and 15, 3 complete ECGs were performed for each subject, and the mean value used as the subject's baseline for each corresponding period.

Both study medications were always administered 1 hour after a medium-fat meal. Tigecycline was administered intravenously (IV) in 0.09% sterile normal saline over 30 minutes for all doses. Digoxin was administered orally with 240 mL of room-temperature water for all doses.

### Period 1

One (1) hour after a medium-fat meal, and after a predose 7-mL blood sample for a baseline tigecycline PK analysis, each subject received a single 100-mg dose of tigecycline.
Subjects received no study medication on days 2 through 5.

### Period 2

On day 6, after a predose 3-mL blood sample and urine samples for baseline digoxin PK analyses, each subject received 0.5 mg of digoxin. On days 8 through 14, each subject received 0.25 mg of digoxin.

### Period 3

On day 15, predose blood samples (5 mL) for determination of digoxin trough levels and blood samples (3 mL) for PK analysis were collected. In addition, a 24-hour urine collection (day 14 to day 15) for PK analysis was completed for each subject. The volume and pH of urine collected during each interval were recorded and an aliquot stored for digoxin analysis.

At approximately 8 AM, each subject received 100 mg of tigecycline. At the same time, each subject received 0.25 mg of digoxin. At approximately 8 PM, each subject received 50 mg of tigecycline.
On days 16 through 18, blood samples for digoxin plasma trough level determination were collected 2 hours before administration of digoxin. Then, at approximately 8 AM, each subject received 0.25 mg of digoxin. In addition, on days 16 through 18, each subject received 50 mg of tigecycline every 12 hours (at approximately 8 AM and 8 PM).

On study day 19 at 8 AM, 1 hour after a medium-fat meal, each subject received 50 mg of tigecycline plus 0.25 mg of digoxin.

### Serum tigecycline determinations

Venous blood samples (7 mL each) for determination of tigecycline concentrations in serum were collected at the following times: on day 1, predose (within 2 hours before the start of the tigecycline infusion), and at 0.5 (end of infusion), 1,1.5,2, 3, 4, 6, 8, 12, 16, 24, 36, 48, 72, and 96 hours after tigecycline administration; and on day 19, predose, and at 0.5 (end of infusion), 1, 1.5, 2, 3, 4, 6, 8, 12, 16, 24, 36, 48, 72, and 96 hours after tigecycline administration.

All samples were collected from an indwelling catheter or by direct venipuncture into blood collection tubes that did not contain any anticoagulant.

Serum tigecycline samples were analyzed by a validated liquid chromatography/tandem mass spectroscopy (LC/MS/MS) method. The standard curve used for serum tigecycline had lower and upper limits of quantitation of 10 and 2000 ng/mL, respectively.

### Serum digoxin determinations

Venous blood samples (3 mL each) for determination of digoxin concentrations in plasma were collected at the following times: on day 7, predose, on day 14 at 0.5, 1, 2, 4, 6, 8, 10, 12, 16, and 24 hours after digoxin administration, and on day 19 at 0.5, 1, 2, 4, 6, 8, 10, 12, 16, and 24 hours after digoxin administration. A serum digoxin sample was collected at hour 0 of day 15. All samples were collected from an indwelling catheter or by direct venipuncture into blood collection tubes containing ethylenediaminetetraacetic acid.
Validated radioimmunoassay (RIA) methods were used for the analysis of digoxin in plasma and urine samples. During validation, the serum RIA assay had a range of 0.150 ng/mL to 8.0 ng/mL and a sensitivity of 0.150 ng/mL.

In addition, digoxin trough samples (5 mL) for determination of digoxin levels (for safety purposes) were routinely collected within 2 hours before digoxin administration on days 10 through 19. This assessment used a commercial microparticle enzyme immunoassay (MEIA, AxSYM Digoxin II assay, Abbott Laboratories, Abbott Park, IL, USA). The reagents for the assay consisted of 6 calibrators (0.0, 0.50, 1.0, 2.0, 3.0, and 4.0 ng/mL) and 3 controls (0.9 [range = 0.6 to 1.2], 1.9 [1.5 to 2.30], and 3.2 [2.60 to 3.8] ng/mL, respectively).

### Urine digoxin determinations

Urine samples for determination of digoxin concentrations were obtained on day 7 within 2 hours before digoxin administration, and on days 14 and 19 before study drug administration, at 0 to 4 hours, 4 to 8 hours, 8 to 12 hours, and 12 to 24 hours after morning digoxin administration. Subjects were required to void completely at the end of the predose period and at the end of each time interval after dose administration to ensure a complete interval collection.

Urine tigecycline was not measured in this study.

### Pharmacokinetic analyses

Pharmacokinetic (PK) parameters for serum tigecycline, plasma digoxin, and urine digoxin were estimated by noncompartmental analysis.( Gibaldi M, Perrier D. Pharmacokinetics. Marcel Dekker, Inc., 1982) Multiple sequential sampling for tigecycline and digoxin over a 96-hour interval during all 3 study periods allowed accurate estimates of PK parameters for both drugs.

Tigecycline peak serum concentration (Cₘₐₓ) and time to peak concentration (tₘₐₓ) were reported from the observed data. Concentrations that were judged to be in the terminal phase were used to obtain the terminal-phase disposition rate constant (λ_{z}) by log-linear regression. The half-life (t_{1/2}) was calculated as 0.693/λ_{z}. Tigecycline concentrations over the time period from hours 24 to 96 were used to estimate the t_{1/2}.

Tigecycline area under the serum concentration-time curve over the 12-hour multiple-dose dose interval (AUC₀₋₁₂ₕ), total area under the concentration-time curve (AUC), peak concentration (Cₘₐₓ), intravenous clearance (CL), mean residence time (MRT), and apparent steady-state volume of distribution (Vₛₛ), were determined. Similarly, plasma digoxin Cₘₐₓ, tₘₐₓ, AUC over the 24-hour dose interval (AUC₀₋₂₄ₕ), and oral-dose clearance (CL/F), together with the percentage of digoxin excreted in urine (Aₑ,%) and digoxin renal clearance (CLᵣ), were also determined.

### Single-dose Tigecycline PK

After a single dose (period 1, days 1 to 5), the area under the concentration-time curve (AUCₜ) and area under the first moment concentration-time curve (AUMCₜ) truncated at the last observable concentration (Cₜ) at time t, were calculated by applying the linear trapezoidal rule to Cₘₐₓ and the log-linear trapezoidal rule thereafter. Total AUC_{0-τ} and AUMC were estimated as follows: AUC = (AUCₜ) + Ct/λ_{z}, and AUMC = (AUMCₜ) + tₗₐₛₜ · Ct/λ_{z} + Ct/λ_{z}•2

The single-dose systemic mean residence time (MRT) was calculated as: MRT = (AUMC/AUC) - T_{inf}/2, where T_{inf} is the infusion time (0.5 hours). The IV clearance (CL) was calculated and normalized by body weight (WT) as follows: CL = Dose/(AUC•WT). The apparent Vₛₛ was estimated by Vₛₛ = CL·MRT.

### Multiple-dose Tigecycline PK

After multiple doses (period 2, day 19), the steady-state AUC (AUC₀₋ₜ) and AUMC (AUMC₀₋ₜ) over the dose interval (t = 12 hours) were also calculated by applying the linear trapezoidal rule to Cₘₐₓ and the log-linear trapezoidal rule thereafter. For this period of the study, the MRT was calculated as: MRT = (AUMC₀₋ₜ + (t·Cₜ/λ_{z}))/AUC₀₋ₜ.

Tigecycline concentrations in individual patients without coadministration of digoxin during period 1 were based on a single 100-mg tigecycline dose, whereas concentrations with coadministration of digoxin during period 2 were based on a 50-mg/12h multiple-dose regimen. According to linear PK theory,( Gibaldi M, Perrier D. Pharmacokinetics. Marcel Dekker, Inc., 1982) the total AUC after a single dose (AUC_{0-∞}) is equal to AUC over the dose interval τ at steady state (AUC_{0 τ}). Therefore, it was possible to determine the effect of digoxin on serum tigecycline exposure by comparing tigecycline AUC_{0-∞} after a single tigecycline dose alone (dose-normalized to 50 mg) with tigecycline AUC_{0-τ} after the concomitant multiple-dose administration of tigecycline and digoxin.

### Digoxin steady-state concentrations

Plasma digoxin steady-state profiles were obtained on study days 14 (period 2, digoxin alone) and 19 (period 3, digoxin with tigecycline). The Cₘₐₓ and tₘₐₓ values were taken directly from the observed data. The λ_{z} and t_{1/2} values were not estimable because blood samples were not collected during the terminal disposition phase. Estimates of the plasma steady-state AUC (AUC₀₋ₜ) on days 14 (period 2) and 19 (period 3) were obtained over 24-hour (AUC₀₋₂₄) intervals.

Digoxin oral-dose clearance and renal clearance

The digoxin oral-dose clearance (CL/F) was calculated and normalized by body weight (WT) as follows: CL/F = Dose/(AUC·WT). Vₛₛ/F and MRT could not be calculated because λ_{z} could not be estimated.

The amount of digoxin excreted in urine over the intervals of 0 to 4, 4 to 8, 8 to 12, and 12 to 24 hours on study days 14 (period 1) and 19 (period 2) were determined in order to estimate the total amount of digoxin excreted in urine (Aₑ,0-24h). The percentage of the dose of digoxin excreted unchanged in urine (Ae,%) was calculated using the formula: Aₑ,% = (Aₑ,0 24h/Dose) · 100. The renal clearance of digoxin (CLᵣ) normalized by body weight (WT) was calculated from the formula: CLᵣ = Aₑ,0-24h/AUC₀₋₂₄ₕ/₂₄ₕ/WT.

### Pharmacodynamic assessments

The pharmacodynamic (PD) analysis was based on changes from baseline in 12-lead ECG parameters (PR, QRS, QT, and QTc intervals, performed at 25 mm/s) at 24 hours after digoxin administration, when serum digoxin concentrations would be expected to be in equilibrium with tissue concentrations. Baseline values for tigecycline alone (period 1) were taken on day 1 just before tigecycline administration, while the baseline values for digoxin alone (period 2) and digoxin + tigecycline (period 3) were taken on day 7 just before the start of digoxin multiple-dose administration.

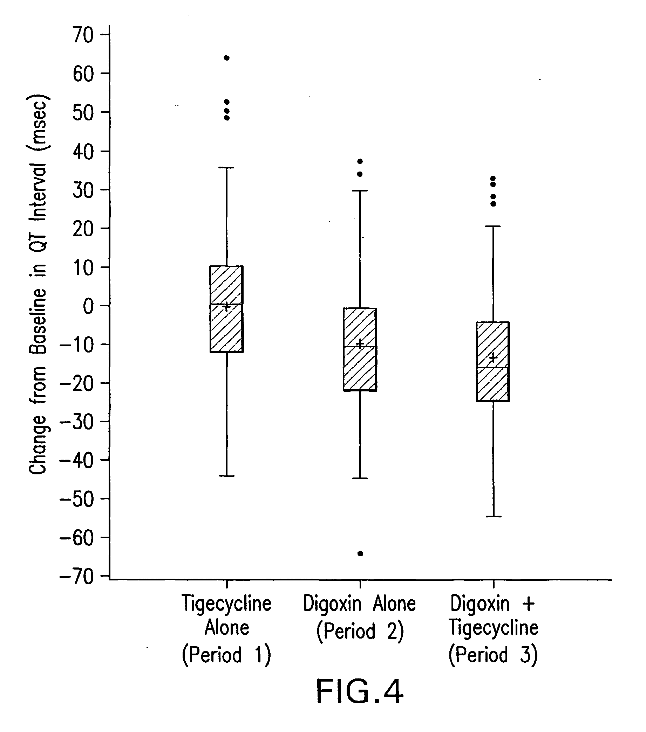

Twelve (12)-lead ECGs were performed at screening, on day-1, on days 1, 7, 14, 15, and 19 within 2 hours before study drug administration, on days 2 through 6, 8 through 13, 16 through 18, 20, 21, and 22, and at the final evaluation at approximately 8 AM. Distribution of ECG changes from predose values in QT interval in healthy subjects. Tigecycline alone, digoxin alone and digoxin + tigecycline concomitantly are shown in Figure 4.

### Statistical analysis

Descriptive statistics were obtained for all demographic characteristics, drug concentrations, PK parameters, and changes from baseline in ECG parameters. Analysis of variance (ANOVA) was performed on the natural logarithm-transformed PK parameters to evaluate treatment and subject effects. An analysis of the change from baseline in ECG parameters with and without multiple-dose tigecycline administration was conducted by using ANOVA, which included terms for subject and treatment effects.

For statistical comparisons, AUC, MRT, and Vₛₛ on day 1 (period 1) were based on concentrations normalized to the 50-mg tigecycline dose given during period 3.

All statistical comparisons for individual PK parameters were performed on log-transformed data. Calculation of statistical power between 2 treatments was based on detecting a 20% difference in log-transformed parameters at the 0.05 significance level.

### Bioequivalence testing

Further comparisons between treatments were performed by using the "two 1-sided tests" bioequivalence procedure for log-transformed data on PK parameters, to determine the equivalence of serum tigecycline PK when tigecycline was given alone and concomitantly with digoxin. Identical equivalence testing was conducted for plasma and urine digoxin.

Geometric least-squares (GLS) mean ratios of tigecycline PK parameters were computed, and their associated 90% confidence intervals (Cls) calculated based on least squares means and the mean square error obtained from the 2-way ANOVA. The test procedure for log-transformed data is equivalent to requiring the ordinary 90% Cls of the geometric least squares (GLS) mean ratio to be in the range of 80% to 120%.( Schuirmann DJ. A comparison of the two one-sided tests procedure and the power approach for assessing the equivalence of average bioavailability. J Pharmacokinet Biopharm 1987;15:657-80) After the log-transformation, these equivalence limits were revised to the customary range of 80% to 125% to allow for symmetry. The SAS statistical software package was used for all statistical analyses.

### Safety evaluations

Safety was evaluated from spontaneously reported signs and symptoms and from the results of physical examinations including weight and height, vital sign measurements, 12-lead ECGs, clinical laboratory evaluations (trough digoxin concentrations, hematology and blood chemistry tests), and routine urinalyses. Adverse events (AEs) were recorded throughout the study.

Digoxin trough samples (5 mL) were collected within 2 hours before administration of digoxin on days 10 through 19.

### RESULTS

Thirty (30) healthy men aged 27-45 years were enrolled. The subjects' demographic characteristics are presented in Table 1.

**TABLE 1. SUBJECT CHARACTERISTICS**

| | Age (y) | Height (cm) | Weight (kg) | Body Mass Index (kg/m²) | Sex | Ethnic Origin | | |
|---|---|---|---|---|---|---|---|---|
| N^{a} | 30.0 | 30.0 | 30.0 | 30.0 | | | | |
| Mean | 36.0 | 181.8 | 82.5 | 25.0 | | | | |
| S.D. | 5.6 | 6.6 | 8.9 | 2.5 | | | | |
| % | | | | | | | | |
| CV | 15.5 | 3.6 | 10.7 | 9.9 | | | Enrolled | Completed |
| Min | 27.0 | 169.5 | 57.7 | 18.9 | (all subjects were men) | Black White Other | 19 | 12 |
| Max | 45.0 | 194.4 | 100.5 | 30.1 | | | 10 | 8 |
| | | | | | | | 1 | 0 |
| | | | | | | | N=30 | N=20 |

| | | | | | | | | |
|---|---|---|---|---|---|---|---|---|
| a: Ten (10) subjects discontinued prematurely from the study and were excluded from all statistical analyses | | | | | | | | |

In the present study, different tigecycline IV dose regimens were used during periods 1 (single dose) and 3 (multiple dose); which prevented a direct comparison of PK parameters obtained from periods 1 and 3. However, since tigecycline exhibits linear pharmacokinetics, based on linear PK theory, (Gibaldi M, Perrier D. Pharmacokinetics. Marcel Dekker, Inc., 1982), it was determined that the following parameters could be compared: (a) total tigecycline exposure (AUC) as reflected by dose-normalized AUC_{0-∞} (period 1) and actual AUC0-12h (period 2), (b) t1/2, CL, and actual AUC₀₋₁₂ₕ for the 2 periods, and (c), MRT and Vₛₛ for the 2 periods, with estimates for period 1 based on concentrations normalized to a 50-mg dose.

### Digoxin Plasma PK2

Tigecycline did not affect the steady-state plasma digoxin AUC₀₋₂₄ₕ, oral-dose CL/F, or digoxin concentrations during the 12- to 24-hour period after dose administration (therapeutic drug monitoring times), although the 90% Cls for Cₘₐₓ and tₘₐₓ fell outside of the equivalence window.

Based on the bioequivalence analysis, 90% Cls for the plasma digoxin AUC₀₋₂₄ₕ and CL/F were both within the 80% to 125% equivalence window, but the 90% Cls for Cₘₐₓ (Cl = 77%-98%) and tₘₐₓ (Cl = 91%-135%) were not. Thus, tigecycline did not affect digoxin total exposure (AUC) or oral-dose clearance (CL/F); but the digoxin absorption rate was slightly decreased.

The descriptive statistics for mean pharmacokinetic parameters for plasma digoxin are presented in Table 2. There were no statistically significant treatment effects for the digoxin PK parameters, although the statistical power was low for Cₘₐₓ (p = 0.067, power = 74%) and tₘₐₓ (p = 0.379, power = 18%).

**TABLE 2. MEAN ± SD PLASMA DIGOXIN PHARMACOKINETIC PARAMETERS (N = 20)**

| Treatment | Statistic | Cₘₐₓ (h) | tₘₐₓ (ng·h/mL) | AUC₀₋₂₄ₕ (ng·h/mL) | CL/F (mL/h/kg) |
|---|---|---|---|---|---|
| Digoxin alone | Mean ± SD | 1.19 ± 0.20 | 1.35 ± 0.56 | 11.7 ± 2.3 | 4.54 ± 1.08 |
| (period 2) | %CV | 17.2 | 41.8 | 19.3 | 23.8 |
| | Geo. Mean | 1.17 | 1.23 | 11.5 | 4.43 |
| | Range | 0.843 - 1.59 | 0.50 - 2.0 | 7.52 - 15.8 | 3.25 - 7.22 |
| | | | | | |
| Digoxin + | Mean ± SD | 1.09 ± 0.46 | 1.48 ± 0.55 | 11.2 ± 2.7 | 4.79 ± 1.21 |
| Tigecycline | %CV | 42.3 | 37.3 | 24.2 | 25.2 |
| (period 3) | Geo. Mean | 1.02 | 1.37 | 10.9 | 4.65 |
| | Range | 0.642 - 2.28 | 0.50 - 2.0 | 7.43 - 17.6 | 3.34-7.11 |
| | | | | | |
| Source | | ----- p-Value from a 2-way ANOVA ----- | | | |
| Subject | | 0.075 | 0.090 | 0.002 | 0.001 |
| Treatment | | 0.067 | 0.379 | 0.272 | 0.272 |
| | | | | | |
| Power | | 0.74 | 0.18 | 0.99 | 0.99 |
| | | | | | |
| | | ----- Two 1-Sided Tests Bioequivalence Procedure ----- | | | |
| GLS Mean | | 87 | 111 | 95 | 105 |
| Ratio | | | | | |
| 90% CI | | 77-98 | 91-135 | 88-103 | 97-113 |

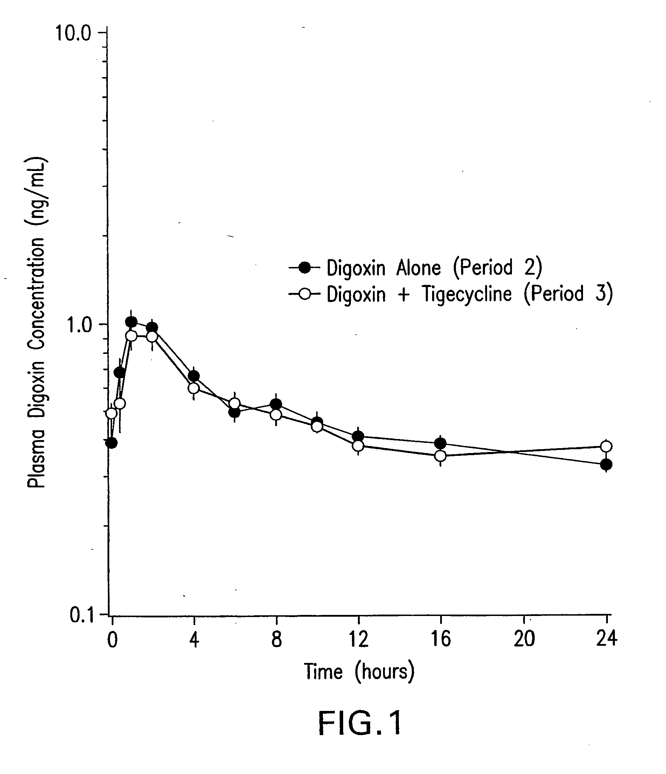

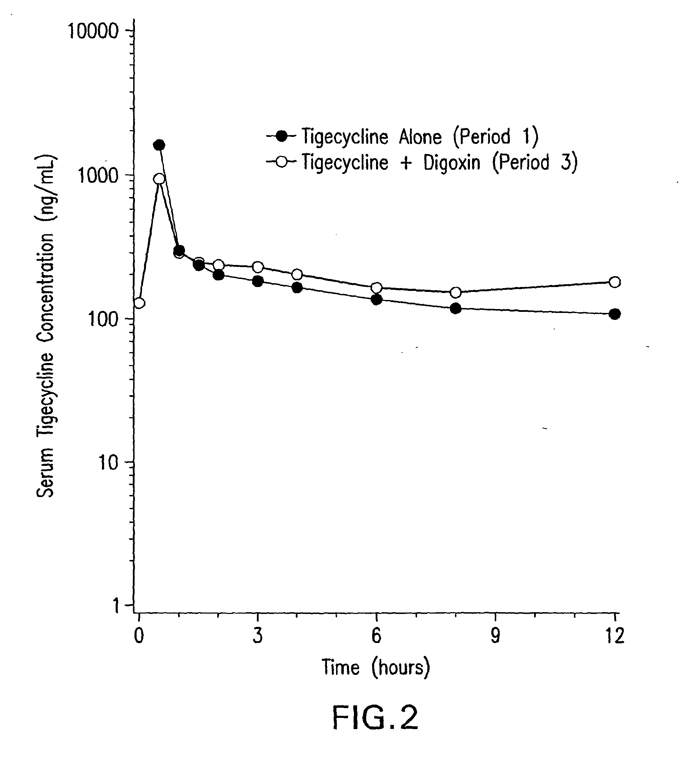

The results of the bioequivalence analysis therefore indicate that tigecycline did not affect the AUC or CL/F of digoxin. Although coadministration of tigecycline decreased the absorption rate of digoxin, as reflected by a concurrent decrease in Cₘₐₓ (13%) and increase in tₘₐₓ (11%), these changes were small and would not be expected to alter the PD effect of the digoxin. In addition, while not being bound by theory as hypothesized tigecycline did not increase the Cmax of digoxin. Furthermore, the 90% Cl for plasma digoxin concentrations at 12, 16 and 24 hours were all within the equivalence window. Mean and individual plasma digoxin concentrations over 24-hour intervals during period 2 (digoxin alone) and period 3 (digoxin plus tigecycline) are presented in Figures 1 and 2_respectively.

### Digoxin Urinary PK

Tigecycline also did not affect the steady-state digoxin urinary PK as shown by measurement of digoxin Aₑ,% and digoxin CLᵣ. Descriptive statistics for urinary digoxin parameters during periods 2 and 3, the results of ANOVA, and the results of the bioequivalence analysis are summarized in Table 3.

**TABLE 3. MEAN ± SD URINARY DIGOXIN PARAMETERS (N = 20)**

| Treatment | Statistic | Aₑ (%) | CLᵣ (mL/min/kg) |
|---|---|---|---|
| Digoxin alone | Mean ± SD | 41.3 ± 9.0 | 1.82 ± 0.37 |
| (period 2) | %CV | 21.8 | 20.2 |
| | Geo. Mean | 40.3 | 1.79 |
| | Range | 24.0 - 60.9 | 1.09 - 2.51 |
| | | | |
| Digoxin + | Mean ± SD | 37.8 ± 9.4 | 1.75 ± 0.40 |
| Tigecycline | %CV | 24.9 | 23.2 |
| (period 3) | Geo. Mean | 36.5 | 1.70 |
| | Range | 19.5 - 50.8 | 0.98 - 2.57 |
| | | | |
| Source | | --- p-Value From a 2-way ANOVA --- | |
| Subject | | 0.110 | 0.005 |
| Treatment | | 0.161 | 0.320 |
| | | | |
| Power | | 0.78 | 0.96 |
| | | | |
| | | Two 1-Sided Tests Bioequivalence Procedure | |
| GLS Mean Ratio | | 91 | 95 |
| 90% CI | | 80-102 | 87-104 |

The results for ANOVA in Table 3 show that there were no statistically significant treatment effects on either total urinary digoxin excretion (p = 0.161) or renal clearance (p = 0.320). Similarly, based on the bioequivalence analysis, 90% Cls for Aₑ,% and digoxin CLᵣ were both within the 80% to 125% equivalence window. Therefore, tigecycline did not affect digoxin urinary PK.

### Tigecycline serum PK

Digoxin did not affect the steady-state AUC, CL, or MRT of tigecycline, although the GLS mean ratios for serum tigecycline t_{1/2} and Vₛₛ fell outside the 80% to 125% equivalence window.
The descriptive statistics for mean pharmacokinetic parameters for serum tigecycline are summarized in Table 4.

**TABLE 4. MEAN ± SD SERUM TIGECYCLINE PHARMACOKINETIC PARAMETERS (N = 20)**

| | | | | | | | |
|---|---|---|---|---|---|---|---|
| | | | | | | | |

| Treatment | Statistic | t_{1/2} (h) | AUC₀₋₁₂ₕ^{a} (ng·h/mL) | AUC^{b,c} (ng·h/mL) | CL (mL/h/kg) | Vss^{c} (L/kg) | MRT^{c} (h) |
|---|---|---|---|---|---|---|---|
| Tigecycline alone | Mean ± SD | 27.7 ± 7.5 | 2480 ± 379 | 2837 ± 732 | 229 ± 56 | 6.53 ± 1.30 | 30.0 ± 8.3 |
| (period 1) | %CV | 27.0 | 15.3 | 25.8 | 24.4 | 19.9 | 27.5 |
| | Geo. Mean | 26.7 | 2452 | 2755 | 222 | 6.43 | 29.0 |
| | Range | 13.5 - 45.0 | 1892 - 3107 | 1810 - 4753 | 135 - 335 | 4.79 -10.41 | 17.1-45.0 |
| | | | | | | | |
| Tigecycline + | Mean ± SD | 40.4 ± 11.9 | 2625 ± 524 | 2625 ± 524 | 243 ± 54 | 8.13 ± 2.68 | 34.4 ± 10.8 |
| Digoxin | %CV | 29.5 | 20.0 | 20.0 | 22.3 | 33.0 | 31.3 |
| (period 3) | Geo. Mean | 38.9 | 2577 | 2577 | 237 | 7.80 | 32.9 |
| | Range | 26.4 - 73.3 | 1843 - 3748 | 1843 - 3748 | 149 - 354 | 5.03 -17.37 | 21.1- 56.7 |
| | | | | | | | |
| Source | | | ----- p-Value from a 2-way ANOVA ----- | | | | |
| Subject | | 0.007 | 0.001 | 0.001 | 0.001 | 0.043 | 0.001 |
| Treatment | | 0.001 | 0.118 | 0.050 | 0.050 | 0.004 | 0.018 |
| | | | | | | | |
| Power | | 0.86 | 1.0 | 1.0 | 1.0 | 0.88 | 0.97 |
| | | | | | | | |
| | | ----- Two 1-Sided Tests Bioequivalence Procedure for Log-Transformed Data -- | | | | | |
| | | | | | | | |
| GLS Mean Ratio | | 146 | 105 | 94 | 107 | 121 | 113 |
| 90% CI | | 131-162 | 100-111 | 88-99 | 101-113 | 109-134 | 104-123 |

| | | | | | | | |
|---|---|---|---|---|---|---|---|
| a: Actual AUC values for both periods 1 and 3. b: AUC = AUC_{0-∞} for tigecycline alone, and AUC = AUC₀₋₁₂ₕ for tigecycline with digoxin. c: The estimates for AUC, MRT, and Vₛₛ during period 1 are based on tigecycline concentrations normalized to a 50-mg dose. | | | | | | | |

Before statistical comparisons, the dose-dependent parameter AUC on day 1 of period 1 was normalized to a 50-mg tigecycline dose. Estimates from the ANOVA were used to compute the geometric least-squares (GLS) ratios and associated 90% Cls for the treatment comparisons.

The results for ANOVA in Table 4 show statistically significant treatment effects for all tigecycline PK parameters except for AUC₀₋₁₂ₕ (p = 0.12, power = 1.0). However, based on the bioequivalence analysis, 90% Cls for the parameters AUC₀₋₁₂ₕ, AUC, CL, and MRT were all within the 80% to 125% equivalence window, but the 90% Cls for t_{1/2} (Cl = 131 % 162%) and Vₛₛ (Cl = 109%-134%) were not within the equivalence window. The results of the bioequivalence analysis therefore indicate that digoxin did not affect the AUC, CL, or MRT of tigecyclirie. Also, because the AUC₀₋₁₂ₕ values on days 1 and 19 were equivalent without normalization for dose, the results indicate that a loading dose of 2 times the maintenance dose reached steady state after the first dose. Although coadministration of tigecycline and digoxin (period 3) increased both tigecycline terminal t_{1/2} and apparent Vₛₛ, these increases did not affect the total exposure or IV clearance of tigecycline

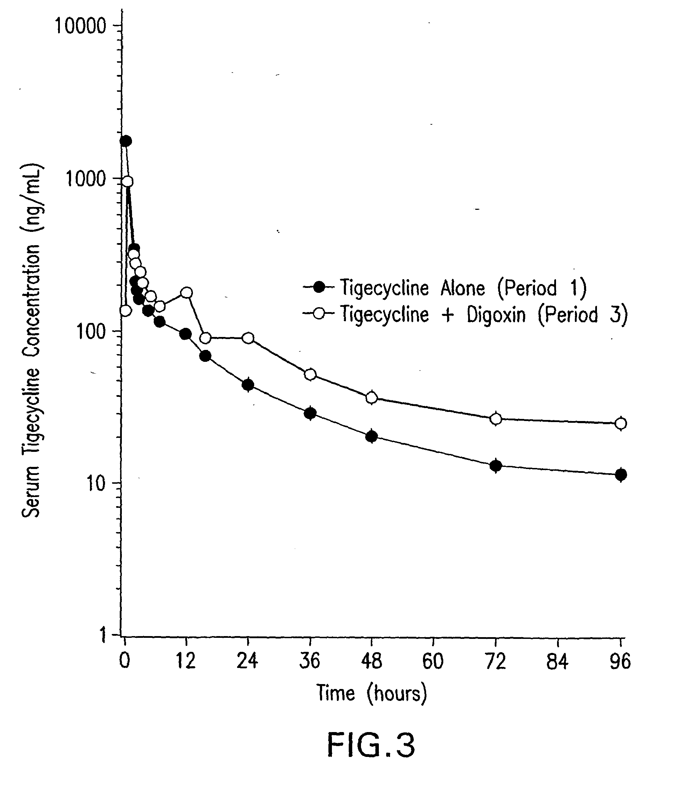

Mean and individual serum tigecycline concentrations over 96 hours during period 1 (tigecycline alone) and period 3 (tigecycline plus digoxin) are presented in Figure 3.

### ECG measurements

Tigecycline did not affect steady-state digoxin pharmacodynamic effects as measured by changes from baseline in ECG parameters.The small concurrent decrease in Cₘₐₓ (13%) and increase in tₘₐₓ (11 %) would not be expected to alter the PD effect of digoxin. Furthermore, the 90% Cls for plasma digoxin concentrations at 12, 16, and 24 hours were all within the equivalence window.

The present study was designed to compare changes from baseline in ECG parameters (PR, QRS, QT, and QTc intervals) at 24 hours after drug administration. At this time point, serum digoxin concentrations would be expected to be in equilibrium with tissue concentration, and the ratio of inotropic response to serum concentrations would be relatively constant. (Reuning RH, Geraets DR. Digoxin. In: Evans WE, Schentag JJ, Jusko WJ, eds. Applied Pharmacokinetics. Spokane: Applied Therapeutics, Inc., 1986:570-623)

Based on ANOVA, there were no significant differences in ECG parameters due to treatment effects at 24 hours after drug administration, except for the QT interval (p = 0.007, period 1 > 2 = 3). The QT interval decreased after digoxin (period 2) compared to tigecycline alone (period 1) but was not changed further when tigecycline was added to digoxin (period 3). These results indicate that coadministration of tigecycline did not produce significant changes in steady-state digoxin PD as measured by changes from baseline in ECG parameters.

### Assay comparisons

It should be noted that the 0-hour samples on days 14 and 19 were analyzed using the MEIA monitoring assay, and the 24-hour samples on these days were analyzed using the RIA PK assay. The mean ± SD ratios for trough samples at 0 and 24 hours (0h/24h) on days 14 and 19 showed values of 22.3% ± 36.0% and 37.4% ± 44.1 %, respectively.

Although the MEIA method was not intended for use in digoxin PK profiling in this study, the hour 0 and hour 24 blood samples for digoxin PK on days 14 and 19 were inadvertently analyzed using this assay. Because the plasma MEIA and plasma digoxin RIA methods had not been cross-validated, it was decided that digoxin concentrations in serum samples from the hour 0 time point on day 15 would be assayed using the serum digoxin RIA method. The resulting data would then permit a comparison of digoxin concentrations at a single time point based on a PK assay (serum digoxin RIA) and monitoring assay (plasma digoxin MEIA). While the 2 assays are based on different biological matrices (plasma as opposed to serum), this difference would not be expected to affect the measured concentrations.

The results presented in Table 5 show that mean ± SD digoxin concentrations measured by the MEIA method were increased by 27.0% ± 24.4% compared with digoxin concentrations measured by the RIA method. The higher digoxin concentrations at 0-hour may be partially because of the use of the MEIA assay.

**TABLE 5. COMPARISON OF DIGOXIN CONCENTRATIONS OBTAINED BY RIA AND MEIA METHODS AT HOUR 0 ON DAY 19**

| | ------------------------ Assay ---------------------- | | (M-R)/M^{c} |
|---|---|---|---|
| Subject | MEIA^{a} | RIA^{b} | (%) |
| 2 | 0.500 | 0.319 | 36.20 |
| 3 | 0.600 | 0.270 | 55.00 |
| 6 | 0.600 | 0.217 | 63.83 |
| 7 | 0.500 | 0.25 | 49.00 |
| 8 | 0.700 | 0.243 | 65.29 |
| 9 | 0.600 | 0.271 | 54.83 |
| 10 | 0.700 | 0.397 | 43.29 |
| 11 | 0.300 | 0.252 | 16.00 |
| 17 | 0.500 | 0.374 | 25.20 |
| 18 | 0.400 | 0.346 | 13.50 |
| 19 | 0.400 | 0.375 | 6.25 |
| 20 | 0.300 | 0.327 | -9.00 |
| 21 | 0.400 | 0.331 | 17.25 |

A statistical comparison (ANOVA) of the concentrations at each time point during periods 2 and 3 is presented in Table 6. The results show that tigecycline did not affect digoxin concentrations at any time point except at 0 hours (p = 0.008) and 24 hours (p = 0.017) after dose administration. The mean ± SD digoxin concentrations at 0 hour (MEIA monitoring assay) and 24 hours (RIA PK assay) on day 19 were increased by 24.9% ± 35.1 % and 16.4% ± 29.5%, respectively, compared to day 14.

**TABLE 6. STATISTICAL COMPARISON OF PLASMA DIGOXIN CONCENTRATIONS FOR SUBJECTS IN PERIODS 2 (DIGOXIN ALONE) AND 3 (TIGECYCLINE + DIGOXIN)**

| | Time After Dose (Hours) | | | | | | | | | | | |
|---|---|---|---|---|---|---|---|---|---|---|---|---|
| Digoxin Alone | | 0 | 0.5 | 1 | 2 | 4 | 6 | 8 | 10 | 12 | 16 | 24 |
| (ng/mL) | Mean | 0.405 | 0.686 | 1.018 | 0.966 | 0.645 | 0.517 | 0.531 | 0.451 | 0.405 | 0.383 | 0.330 |
| | S.D. | 0.094 | 0.426 | 0.375 | 0.193 | 0.126 | 0.107 | 0.104 | 0.092 | 0.083 | 0.100 | 0.090 |
| | % | 23.3 | 62.1 | 36.9 | 20.0 | 19.5 | 20.6 | 19.5 | 20.3 | 20.5 | 26.3 | 27.3 |
| | CV | | | | | | | | | | | |
| | N | 20 | 20 | 20 | 20 | 20 | 20 | 20 | 20 | 20 | 20 | 20 |
| | Min | 0.300 | 0.227 | 0.254 | 0.493 | 0.400 | 0.320 | 0.362 | 0.265 | 0.235 | 0.206 | 0.188 |
| | Max | 0.600 | 1.430 | 1.590 | 1.310 | 0.847 | 0.664 | 0.702 | 0.579 | 0.544 | 0.560 | 0.584 |
| Digoxin+Tigecycline | Mean | 0.491 | 0.532 | 0.910 | 0.901 | 0.600 | 0.534 | 0.491 | 0.448 | 0.385 | 0.347 | 0.372 |
| (ng/mL) | S.D. | 0.124 | 0.494 | 0.474 | 0.240 | 0.148 | 0.176 | 0.111 | 0.107 | 0.108 | 0.104 | 0.092 |
| | % | 25.4 | 92.9 | 52.1 | 26.7 | 24.6 | 33.0 | 22.5 | 23.9 | 28.0 | 30.0 | 24.6 |
| | CV | | | | | | | | | | | |
| | N | 20 | 20 | 20 | 20 | 20 | 20 | 20 | 20 | 20 | 20 | 20 |
| | Min | 0.300 | 0.184 | 0.299 | 0.533 | 0.370 | 0.297 | 0.335 | 0.288 | 0.221 | 0.224 | 0.206 |
| | Max | 0.700 | 2.280 | 2.280 | 1.560 | 0.934 | 0.969 | 0.679 | 0.691 | 0.631 | 0.594 | 0.589 |

| | P-Values From Analysis of Variance | | | | | | | | | | | |
|---|---|---|---|---|---|---|---|---|---|---|---|---|
| Source of Variation | | | | | | | | | | | | |
| Subject | | 0.049 | 0.152 | 0.053 | 0.019 | 0.019 | 0.046 | 0.029 | 0.028 | 0.213 | 0.002 | 0.001 |
| Treatment | | 0.008 | 0.114 | 0.275 | 0.176 | 0.146 | 0.908 | 0.122 | 0.840 | 0.378 | 0.087 | 0.017 |
| Statistical Power (%) | | 84 | 0 | 14 | 90 | 94 | 80 | 95 | 92 | 74 | 89 | 97 |

### Tolerability

No deaths, serious adverse events (SAEs), or clinically important changes in laboratory values or vital signs occurred during this study.

Ten (10) subjects withdrew from the study; 9 did so because of AEs. Twenty-nine (29) of 30 subjects (96.7%) reported at least 1 treatment-emergent adverse event (TEAE). The most frequently reported (≥10%) treatment-related TEAEs occurred during period 3 (tigecycline + digoxin): nausea (83%), dyspepsia (28%), headache (24%), vomiting (24%), injection site reaction (21 %) and injection site phlebitis (21 %), abdominal pain (14%), anorexia (17%), diarrhea (10%), dizziness (10%), insomnia (10%), and taste perversion (10%).

All 9 subjects who withdrew from the study did so during period 3; 4 subjects withdrew because of vomiting and 3 withdrew because of nausea. One (1) subject withdrew because of myalgia (musculoskeletal chest pain) of moderate intensity. One (1) subject withdrew because of a worsening of a first-degree atrioventricular block that was not detected at screening; this was judged by the investigator to be related to treatment with digoxin.

## Claims

1. Use of tigecycline in combination with digoxin in the preparation of a medicament for treating or preventing a bacterial infection and a cardiac insufficiency condition in a human.

2. Use of tigecycline in combination with digoxin in the preparation of a medicament for controlling from increasing steady state digoxin plasma levels in a human experiencing cardiac insufficiency condition and a bacterial infection.

3. Use of tigecycline and digoxin in the preparation of a medicament for treating, controlling or reducing the risk of a bacterial infection and a cardiac insufficiency condition in a human.

4. Use of tigecycline and digoxin in the preparation of a medicament for treating, controlling or reducing the risk of a cardiac insufficiency condition and a bacterial infection in a human.

5. Use of tigecycline in the preparation of a medicament for treating, controlling or reducing the risk of a bacterial infection in a human undergoing treatment with digoxin.

6. Use of tigecycline in the preparation of a medicament for treating or preventing a bacterial infection in a human which treatment also comprises administration of digoxin for cardiac insufficiency.

7. Use of digoxin in the preparation of a medicament for treating or preventing cardiac insufficiency in a human undergoing treatment with tigecyclin.

8. Use of digoxin in the preparation of a medicament for treating or preventing cardiac insufficiency in a human which treatment also comprises administration of tigecycline for treatment or preventing bacterial infection.

9. A product comprising tigecycline and digoxin as a combined preparation for simultaneous, sequential or separate use in the treatment or prevention of a bacterial infection and a cardiac insufficiency in a human.

## Patentansprüche

1. Verwendung von Tigecyclin in Kombination mit Digoxin bei der Herstellung eines Arzneimittels zur Behandlung oder Prävention einer bakteriellen Infektion und einer Herzinsuffizienz in einem Menschen.

2. Verwendung von Tigecyclin in Kombination mit Digoxin bei der Herstellung eines Arzneimittels zur Kontrolle von ansteigenden Digoxin-Plasma-Steady-State-Spiegeln in einem Menschen, der an einer Herzinsuffizienz und einer bakteriellen Infektion leidet.

3. Verwendung von Tigecyclin und Digoxin bei der Herstellung eines Arzneimittels zur Behandlung, Kontrolle oder Verringerung des Risikos einer bakteriellen Infektion und einer Herzinsuffizienz in einem Menschen.

4. Verwendung von Tigecyclin und Digoxin bei der Herstellung eines Arzneimittels zur Behandlung, Kontrolle oder Verringerung des Risikos einer Herzinsuffizienz und einer bakteriellen Infektion in einem Menschen.

5. Verwendung von Tigecyclin bei der Herstellung eines Arzneimittels zur Behandlung, Kontrolle oder Verringerung des Risikos einer bakteriellen Infektion in einem Menschen, der einer. Behandlung mit Digoxin unterzogen wird.

6. Verwendung von Tigecyclin bei der Herstellung eines Arzneimittels zur Behandlung oder Prävention einer bakteriellen Infektion in einem Menschen, wobei diese Behandlung außerdem die Verabreichung von Digoxin gegen Herzinsuffizienz umfasst.

7. Verwendung von Digoxin bei der Herstellung eines Arzneimittels zur Behandlung oder Prävention von Herzinsuffizienz in einem Menschen, der einer Behandlung mit Tigecyclin unterzogen wird.

8. Verwendung von Digoxin bei der Herstellung eines Arzneimittels zur Behandlung oder Prävention von Herzinsuffizienz in einem Menschen, wobei diese Behandlung außerdem die Verabreichung von Tigecyclin zur Behandlung oder Prävention einer bakteriellen Infektion umfasst.

9. Produkt, das Tigecyclin und Digoxin als ein Kombinationspräparat zur gleichzeitigen, sequentiellen oder separaten Verwendung bei der Behandlung oder Prävention einer bakteriellen Infektion und einer Herzinsuffizienz in einem Menschen umfasst.

## Revendications

1. Utilisation due tigécycline en combinaison avec de la digoxine dans la préparation d'un médicament pour traiter ou prévenir une infection bactérienne et un état d'insuffisance cardiaque chez un humain.

2. Utilisation de tigécycline en combinaison avec de la digoxine dans la préparation d'un médicament pour la maîtrise de l'augmentation de niveaux dans le plasma de digoxine à l'équilibre chez un humain souffrant d'un état d'insuffisance cardiaque et d'une infection bactérienne.

3. Utilisation de tigécycline et de digoxine dans la préparation d'un médicament pour traiter, maîtriser ou réduire le risque d'une infection bactérienne et d'un état d'insuffisance cardiaque chez un humain.

4. Utilisation de tigécycline et de digoxine dans la préparation d'un médicament pour traiter, maîtriser ou réduire le risque d'un état d'insuffisance cardiaque et d'une infection bactérienne chez un humain.

5. Utilisation de tigécycline dans la préparation d'un médicament pour traiter, maîtriser ou réduire le risque d'une infection bactérienne chez un humain recevant un traitement à base de digoxine.

6. Utilisation de tigécycline dans la préparation d'un médicament pour traiter ou prévenir une infection bactérienne chez un humain, lequel traitement comprend également l'administration de digoxine pour une insuffisance cardiaque.

7. Utilisation de digoxine dans la préparation d'un médicament pour traiter ou prévenir une insuffisance cardiaque chez un humain recevant un traitement à base de tigécycline.

8. Utilisation de digoxine dans la préparation d'un médicament pour traiter ou prévenir une insuffisance cardiaque chez un humain, lequel traitement comprend également l'administration de tigécycline pour le traitement ou la prévention d'une infection bactérienne.

9. Produit comprenant de la tigécycline et de la digoxine en tant que préparation combinée pour une utilisation simultanée, séquentielle ou séparée dans le traitement ou la prévention d'une infection bactérienne et d'une insuffisance cardiaque chez un humain.
